# EUROPÄISCHE PATENTSCHRIFT

(11) **EP 2 682 113 B1**
(45) Veröffentlichungstag und Bekanntmachung des Hinweises auf die Patenterteilung: **31.10.2018**
(21) Anmeldenummer: 12174672.1
(22) Anmeldetag: 02.07.2012
(51) Int. Cl.: A61K 9/70

(54) **Verschließen wirkstoffhaltiger Laminate**
Sealing laminates containing agents
Fermeture de laminé contenant une substance active

(43) Veröffentlichungstag der Anmeldung: 08.01.2014
(73) Patentinhaber: Luye Pharma AG, 83714 Miesbach (DE)
(72) Erfinder: Dabdoub, Nicola, 80933 München (DE); Antropius, Peter, 83666 Schaftlach (DE); Beckert, Thomas, 88447 Warthausen (DE); Salman, Nouha, 81272 München (DE); Schneider, Lorena, 83043 Bad Aibling (DE); Schurad, Björn, 81667 München (DE)
(74) Vertreter: Meinken Zehetner Patentanwälte

(56) Entgegenhaltungen:
- EP-A1- 0 556 158
- EP-A1- 0 577 622
- EP-A2- 1 612 158
- WO-A2-2012/089811
- DE-A1- 3 642 600
- DE-T2- 3 586 707
- US-A- 5 662 926
- US-A- 5 902 433
- US-A1- 2010 086 582
- Dichte Verbindungen: "Kunststoffschweißen mit Hochleistungsdiodenlaser", Jahrg, 1 January 2004 (2004-01-01), 6 March 2018 (2018-03-06), XP055457201, Retrieved from the Internet: URL:http://www.plastverarbeiter.de/wp-cont ent/uploads/migrated/docs/1039_29371.pdf [retrieved on 2018-03-06]
- NEIL BROWN ET AL: "Non-contact laser sealing of thin polyester food packaging films", OPTICS AND LASERS IN ENGINEERING, ELSEVIER, AMSTERDAM, NL, vol. 50, no. 10, 2 April 2012 (2012-04-02) , pages 1466-1473, XP028500061, ISSN: 0143-8166, DOI: 10.1016/J.OPTLASENG.2012.04.001 [retrieved on 2012-05-15]
- S. Michel: "Kunststoffe mit 3D-Laserschweißen schonend verbinden", Konstruktionspraxis, 21 September 2007 (2007-09-21), pages 1-6, XP055457703, Retrieved from the Internet: URL:https://www.konstruktionspraxis.vogel. de/kunststoffe-mit-3d-laserschweissen-scho nend-verbinden-a-114206/ [retrieved on 2018-03-08]

## Beschreibung

Die vorliegende Erfindung betrifft ein transdermales Applikationssystem und bezieht sich im Besonderen auf langzeitlagerfähige transdermale Applikationssysteme sowie die Halbzeuge zu deren Herstellung, bei denen ein effektiver Einschluss der Wirkstoffe und der sie enthaltenden Matrix im System auch während längerer Lagerungszeiten gewährleistet ist.

Transdermale Applikationssysteme (Akronym TDS von englisch Transdermal Delivery System) dienen dem Verabreichen von Arzneistoffen durch die Haut eines Patienten in dessen Blutkreislauf. Gegenwärtig sind zwei Grundtypen transdermaler therapeutischer Systeme bekannt, Reservoirsysteme und Matrixsysteme. Bei den sogenannten Reservoirsystemen ist der Wirkstoff in einem flüssigen, halbfesten oder festen Reservoir enthalten, wobei die Wirkstoffabgabe mittels einer Membran reguliert wird. Bei den sogenannten Matrixsystemen ist der Wirkstoff in einer nichtflüssigen Polymermatrix enthalten, wobei der Wirkstoffeintrag in den Blutkreislauf eines Patienten durch Diffusion des oder der Wirkstoffe entlang des Wirkstoffkonzentrationsgefälles, das zwischen der die Haut kontaktierenden Applikationsseite der Matrix und dem vom Blutgefäßsystem durchsetzten Bereich der Haut besteht, erfolgt.

Der erzielbare Wirkstoffblutspiegel wird, abgesehen von der Auflagefläche der Matrix auf der Haut des Patienten, im Wesentlichen über die Konzentration des Wirkstoffs in der Matrix bestimmt, die der beabsichtigten Verabreichungsdauer entsprechend hoch gewählt sein muss. Zur besseren Handhabung und auch um einen direkten Kontakt der wirkstoffhaltigen Matrix mit der Haut zu gewährleisten, ist die Matrix zumeist aus einem selbsthaftenden Polymer gebildet.

Um der Gefahr einer ungewollten Kontamination von mit dem TDS in Berührung kommenden Personen oder Gegenständen vorzubeugen, ist die wirkstoffhaltige Matrix üblicherweise zwischen einer wirkstoffundurchlässigen Rückschicht und einer ebenfalls wirkstoffundurchlässigen abziehbaren Schutzfolie angeordnet. Die auch als Backingfolie bezeichnete Rückschicht bedeckt dabei die nicht zum Kontakt mit der Haut eines Patienten vorgesehene Rückseite der Matrix, die Schutzfolie die zum Kontakt mit der Haut eines Patienten vorgesehene Applikationsseite. Die Schutzfolie dient in erster Linie dem Schutz der Matrix vor Verschmutzung und dem Schutz von Personen und Umgebung vor ungewollter Kontamination mit dem in der Matrix enthaltenen Wirkstoff.

Die Patentschrift US 5 662 926 A ist mit transdermalen therapeutischen Systemen befasst, die in Form eines Laminats (Figur 8) ausgebildet sind, bei dem eine wirkstoffhaltige Matrix zwischen einer Rückschicht und einer Schutzschicht angeordnet ist, wobei die Rückschicht und die Schutzschicht über eine Heißversiegelung verbunden werden können, um eine vorzeitige Migration oder ein Vermischen verschiedener Wirkstoffe in den beiden Matrices vorzubeugen.

In der Patentanmeldung EP 0 556 158 A1 wird ein Verfahren zum Versiegeln eines transdermalen Applikationssystems offenbart, bei dem die das wirkstoffhaltige Element des Systems einfassenden Folien mithilfe eines Ultraschallschweißverfahrens außerhalb des Elements miteinander verschweißt werden.

In DE 35 86 707 T2 wird ein transdermales Applikationssystem offenbart, bei dem eine wirkstoffhaltige bahnförmige Plastisolschicht auf die Selbstklebeschicht einer Rückseitenbahn aufgebracht ist, wobei sich die Selbstklebeschicht über die gesamte Breite der Rückseitenbahn erstreckt und die Plastisolschicht, die schmäler als die Rückseitenbahn ist, so auf der Rückseitenbahn angeordnet ist, dass die Klebeschicht zu beiden Seiten der Plastisolbahn freiliegt. Eine Trennpapierbahn von der Breite der Rückseite deckt schließlich die freiliegende Klebeschicht und die Plastisolschicht ab.
Neil Brown et al.: "Non-contact laser sealing of thin polyester food packaging films", Optics and Lasers in Engineering Bd. 50, Nr. 10, 2012, S. 1466-1473 beschreiben das kontaktlose Versiegeln eines PET-Behälters mit einer dünnen Verschlussfolie auf PET-Basis für die Nahrungsmittelverpackung. Die Versiegelung erfolgt mit Hilfe der mit Hilfe eines Lasertransmissionsverfahrens.

Bei gängigen Verfahren zur Herstellung von transdermalen therapeutischen Matrixsystemen wird das wirkstoffhaltige Matrixmaterial zunächst auf eine wirkstoffundurchlässige Trägerfolie aufgebracht und anschließend mit einer ebenfalls wirkstoffundurchlässigen Abdeckfolie bedeckt. In der Regel sind Trägerfolie und Abdeckfolie bahnförmig ausgebildet, worunter zu verstehen ist, dass die Folien eine bestimmte Breite aufweisen, ihre die Breite um ein Vielfaches übersteigende Länge jedoch nicht näher spezifiziert ist. Das Aufbringen des Matrixmaterials erfolgt üblicherweise ebenfalls bahnförmig, so dass ein Laminat aus Trägerfolie, Abdeckfolie und dazwischen angeordneter Matrixlage entsteht. Das Laminat wird meist auf einen Spulenkörper aufgewickelt, da dessen Weiterverarbeitung zu einzelnen transdermalen therapeutischen Systemen üblicherweise zeitlich und räumlich getrennt von dessen Herstellung stattfindet.

Die genaue Zusammensetzung des Matrixmaterials richtet sich nach dem bzw. den zu verabreichenden Wirkstoffen.

Als zur Ausbildung einer Matrix geeignete Materialien sind beispielsweise Homo- und Copolymere von (Meth)acrylaten, Polyvinylether, Polyisobutylene, Polyisoprenkautschuk, Styrol-Butadien-Copolymere, Styrol-Butadien-Styrol-Copolymere bekannt. Von den (Meth)acrylat-Copolymeren können beispielsweise die Copolymere von Alkylacrylaten und/oder Alkylmethacrylaten und weiteren ungesättigten Monomeren genannt werden, wie Acrylsäure, Methacrylsäure, Acrylamid, Dimethylacrylamind, Dimethylaminoethylacrylamid, Acrylnitril und/oder Vinylacetat. Die genaue Zusammensetzung eines Matrixmaterials richtet sich jeweils nach dem mit dem Matrixsystem zu verabreichenden Wirkstoff und eventuell hierfür weiter erforderlichen Stoffen, wie zum Beispiel Permeationsförderer.

Die Viskosität von einigen dieser wirkstoffhaltigen Matrices ist bei Raumtemperatur (18 bis 22 °C) und auch darunter oft bereits so gering, dass diese nicht mehr formstabil sind und zu fließen beginnen. Dieses Fließverhalten wird als kalter Fluss bezeichnet und führt dazu, dass wirkstoffhaltiges Matrixmaterial an den Seitenrändern des Laminats aus diesem austreten kann.

Insbesondere, wenn das Laminat bis zur Weiterverarbeitung längere Zeit zwischengelagert wird, kann der durch den kalten Fluss verursachte Matrixaustritt zu einer Verringerung der auf die Fläche bezogenen Wirkstoffkonzentration in Richtung der Randzonen des Laminats führen. Transdermale therapeutische Applikationssysteme, die aus Gebieten nahe den Rändern des Laminats gewonnen werden, weisen somit eine geringere Wirkstoffflächenkonzentration auf, als solche aus zentraleren Gebieten des Laminats. In der Folge können die vorgesehenen Wirkstoffblutspiegel bei einer Applikation solcher Systeme unter Umständen nicht mehr erreicht werden.

Da der reale Wirkstoffgehalt bei transdermalen therapeutischen Systemen den gewünschten Nominalgehalt nicht unterschreiten sollte, muss die Weiterverarbeitung von Laminaten mit kalt fließenden Matrices somit innerhalb kurzer Zeitspannen erfolgen. Eine kostengünstige Vorratsproduktion an Laminat ist bei Verwendung kalt fließender Matrices nicht möglich. Dies stellt wiederum erhöhte Anforderungen an die Logistik des Produktionsprozesses und führt in der Regel, da sich die Menge des jeweils herzustellenden Laminats an der Anzahl der aktuell herzustellenden transdermalen therapeutischen Systeme orientiert, zu einer unökonomischen Ausnutzung von Herstellungsanlagen und einer ineffizienten Nutzung der Rohmaterialien.

Doch auch bei fertig konfektionierten transdermalen therapeutischen Systemen treten bei Verwendung kalt fließender Matrices Probleme auf. Es hat sich gezeigt, dass kalt fließende Matrices über die Randzonen von Schutz- und Backingfolie in die Umgebung eines transdermalen therapeutischen Systems austreten können und die das jeweilige System aufnehmende Verpackung kontaminieren. Hierdurch entsteht eine erhebliche Kontaminationsgefahr für Dritte, sowohl beim Verabreichen als auch beim Tragen eines solchen TDS. Außerdem besteht auch hier die Gefahr, dass die auf die Applikationsfläche bezogene Wirkstoffkonzentration, allgemein als Flächengewicht des Wirkstoffs bezeichnet, so weit gesunken ist, dass die vorgesehenen Wirkstoffblutspiegel und damit die Therapieziele nicht mehr erreicht werden.

Es ist daher wünschenswert, ein als Halbzeug für die Herstellung von transdermalen therapeutischen Systemen verwendbares Laminat sowie transdermale therapeutische Systeme so auszubilden, dass ein Austreten einer kaltfließenden Matrix aus dem Laminat bzw. den transdermalen therapeutischen Systemen nicht mehr möglich ist.

Diese Aufgabe wird erfindungsgemäß durch die Merkmale der unabhängigen Patentansprüche gelöst.

Entsprechende Ausführungsformen solcher Laminate und transdermaler therapeutischer Systeme weisen eine erste wirkstoffundurchlässige Folie, eine zweite wirkstoffundurchlässige Folie und eine zwischen der ersten und der zweiten Folie angeordnete wirkstoffhaltige Matrix auf, wobei erste und zweite Folie über die Ränder der Matrix hinausreichen und in Bereichen außerhalb der Matrix so formschlüssig miteinander verbunden sind, dass die Matrix die Verbindung von erster und zweiter Folie nicht durchdringen kann, wobei die Festigkeit der Verbindung von erster und zweiter Folie geringer ist als die Materialfestigkeit von sowohl der ersten als auch der zweiten Folie.

Solche Ausführungsformen weisen den Vorteil auf, dass die beiden Folien zumindest an den Stellen über die Matrix hinausreichen und dort miteinander verbunden werden, an denen ein Verfließen des Matrixmaterials auftreten kann.

Da die Festigkeit der Verbindung von erster und zweiter Folie geringer als die Materialfestigkeit von sowohl der ersten als auch der zweiten Folie ist, kann die Verbindung bei Bedarf aufgetrennt werden, ohne dass eine der beiden Folien einreißt.

Die stoffschlüssige Verbindung von erster und zweiter Folie kann auch als Schweißverbindung ausgebildet sein (nicht erfindungsgemäß), wobei die Schweißverbindung sowohl als direkte Verschweißung von erster und zweiter Folie, als auch als Verschweißung einer zwischen erster und zweiter Folie angeordneten Zwischenfolie mit der ersten und mit der zweiten Folie ausgebildet sein kann. Die Schweißverbindung unter Verwendung einer Zwischenfolie ist insbesondere in Verbindung mit dicken Matrices aber auch bei steifen Folien von Vorteil, da sie einem Zusammendrücken der Matrix und einer damit verbundenen Kissenbildung, bei der sich das Flächengewicht der Matrix mit dem Abstand zu deren Randzone verändert, wirksam vorbeugt.

Die Schweißverbindung kann als Ultraschallschweißverbindung ausgebildet. Als Ultraschallschweißverbindung wird eine mittels Ultraschallschweißen erstellte Materialverbindung bezeichnet. Eine solche Schweißverbindung zeichnet sich insbesondere dadurch aus, dass die Grundeigenschaften der verschweißten Materialien gegenüber denen der nicht verschweißten Materialien unverändert sind. Dies ist in erster Linie eine Folge der geringen thermischen Belastung der Materialien beim Schweißvorgang, die ihrerseits auf der durch den Effekt der Molekular- und Grenzflächenreibung bedingten Konzentration der eingebrachten Energie auf die sich berührenden Grenzflächen beruht. Da das Material im Fügebereich beim Ultraschallschweißen, anders als beim thermischen Schweißen, nicht durchgehend erhitzt wird, besteht keine Gefahr einer Zersetzung von Matrixmaterial bzw. einer Zersetzung oder Veränderung von Wirkstoffen in der Nähe der Schweißverbindung.

Außerdem kann die Schweißverbindung durch das Transmissions-Laserschweißverfahren ausgebildet werden, bei dem mithilfe eines NIR-Lasers (Laser mit einer Emissionswellenlänge im nahen Infrarotbereich) Material an der Kontaktfläche von erster und zweiter Folien bzw. an den Kontaktflächen der Zwischenfolie mit jeweils der ersten und der zweiten Folie lokal erweicht wird. Die Konzentration des Energieeintrags auf die Kontaktflächen beruht auf der äußerst geringen Absorptionsfähigkeit der Folienmaterialien für Licht im nahen Infrarot. Die lokale Absorption des Laserlichts an der Kontaktfläche wird durch Beschichten der Kontaktfläche von zumindest einer der sich jeweils berührenden Folien mit einer dünnen Absorberschicht bewirkt. Da beim Transmissions-Laserschweißen nur der Bereich unmittelbar an der Kontaktfläche erweicht wird, ist der zum Erstellen einer Schweißverbindung erforderliche Energieeintrag des Lasers in die Folien minimal, so dass die Grundeigenschaften der verschweißten Materialien gegenüber denen der nicht verschweißten Materialien unverändert oder zumindest nahezu unverändert sind. Bei Verwendung einer Zwischenfolie ist die Auftragsdicke der Absorberschicht bei bevorzugten Ausführungsformen so an deren NIR-Absorptionskoeffizienten abgestimmt, dass bei einseitiger Durchstrahlung des Folienverbunds an jeder der beiden Kontaktflächen eine vergleichbare Energiemenge absorbiert wird.

Der Stoffschluss von erster und zweiter Folie kann in Form mehrerer inselförmiger Verbindungen, wie beispielsweise in Form sich nicht berührender Verbindungspunkte, realisiert werden, deren Abstand zueinander so gewählt ist, dass kein Bestandteil der wirkstoffhaltigen Matrix zwischen den inselförmigen Verbindungen hindurchkriechen kann. Eine gezielte Einstellung einer zum Trennen der Verbindung erforderlichen Zugkraft ist so möglich.

Zum Formschluss weist eine der beiden Folien bei vorteilhaften Ausführungsformen Prägemuster auf, deren Musterelemente in entsprechend ausgebildete Prägemusterelemente der anderen der beiden Folien so formschlüssig eingreifen, dass eine Bewegung der Folien lateral zueinander unterbunden ist und der zum Trennen der Folien senkrecht zu deren lateraler Ausdehnung erforderliche Zug größer als die durch das Eigengewicht der Komponenten des Verbundes auf die formschlüssige Verbindung ausgeübte Kraft ist. Ein entsprechender Formschluss wird vorzugsweise mithilfe eines Rändelverfahrens realisiert. Unter lateral ist hierbei eine Richtung parallel zu einer der Hauptflächen der Folien zu verstehen, die bezogen auf eine der Folien jeweils durch deren Dicke beabstandet sind.

Bei alternativen Ausführungsformen ist der Formschluss zwischen erster und zweiter Folie mittels einer zwischen erster und zweiter Folie angeordneten streifen- oder gitterförmigen Zwischenfolie realisiert, in der Prägemuster ausgebildet sind, die in bzw. in die entsprechend ausgebildete Prägemuster der beiden Folien so formschlüssig eingreifen, dass eine Bewegung der Folien lateral zueinander unterbunden ist und der zum Trennen der Folien senkrecht zu deren lateraler Ausdehnung erforderliche Zug größer als das Flächengewicht des Verbunds aus erster Folie, zweiter Folie, Zwischenfolie und Matrix ist. Die Verwendung von Zwischenfolien ist insbesondere in Verbindung mit dicken Matrices aber auch bei steifen Folien von Vorteil, da sie einem Zusammendrücken der Matrix und einer damit verbundenen Kissenbildung, bei der sich das Flächengewicht der Matrix mit dem Abstand zu deren Randzone verändert, wirksam vorbeugt.

Die Festigkeit der formschlüssigen Verbindung ist geringer als die Festigkeit der ersten und die Festigkeit der zweiten Folie.

Weitere Merkmale der Erfindung ergeben sich aus der nachfolgenden Beschreibung von Ausführungsbeispielen in Verbindung mit den Ansprüchen sowie den Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung einiger Ausführungsbeispiele der Erfindung wird auf die beiliegenden Figuren Bezug genommen, von denen
- Figur 1: eine schematische Darstellung einer Vorrichtung zur Herstellung eines Laminats aus Trägerfolie, wirkstoffhaltiger Matrix und Abdeckfolie mit Verschluss der Folien außerhalb des Matrixmaterials zeigt,
- Figur 2: die Vorrichtung von Figur 1 in einer schematischen Draufsicht für ein Laminat mit einer einzelnen Matrixbahn illustriert,
- Figur 3: die Vorrichtung von Figur 1 in einer schematischen Draufsicht für ein Laminat mit mehreren Matrixbahnen illustriert,
- Figur 4: einen Querschnitt durch das Laminat von Figur 3 in einer schematisierten Querschnittsansicht zeigt,
- Figur 5: einen Querschnitt durch das Laminat von Figur 3 nach einem Kaltverfließen des Matrixmaterials zeigt,
- Figur 6: den Einschluss des Matrixmaterials durch Verbinden von Trägerfolie und Abdeckfolie in einer schematischen Querschnittsdarstellung veranschaulicht,
- Figur 7: ein Laminat mit mehreren Matrixinseln in einer schematischen Draufsicht veranschaulicht, und
- Figur 8: die Verbindung von Trägerfolie und Abdeckfolie vermittels einer zwischen den Matrixmaterialien angeordneten Zwischenfolie schematisch veranschaulicht.

In den Figuren sind Elemente, die im Wesentlichen gleiche technische Funktionen erfüllen, mit gleichen Bezugszeichen versehen. Verschiedene Ausgestaltungen dieser Elemente weisen ähnliche Bezugszeichen auf.

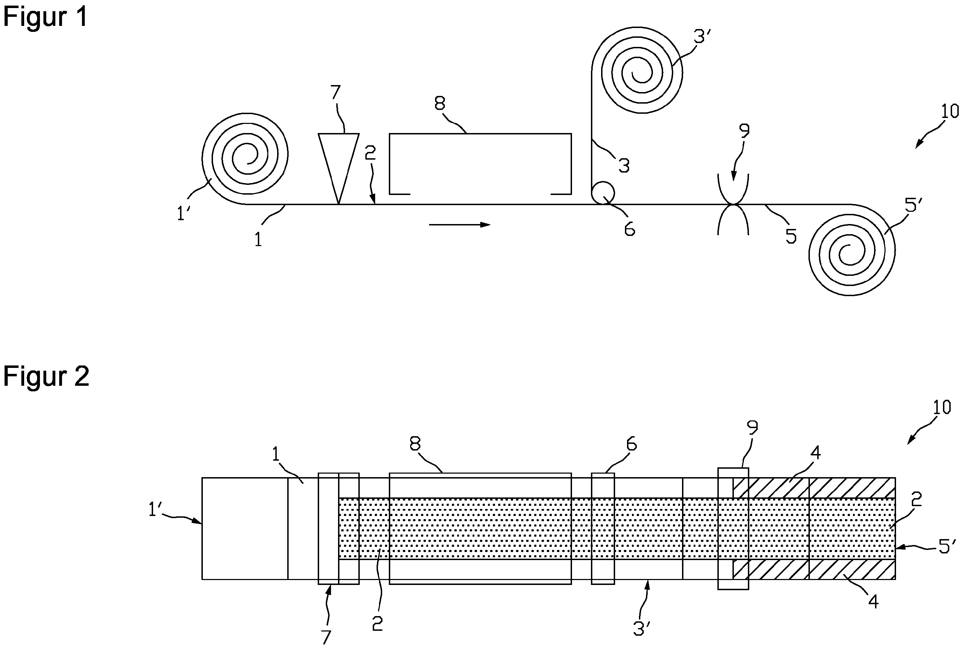

Die schematische Darstellung von Figur 1 veranschaulicht eine Vorrichtung 10 zur Herstellung von Laminaten 5, die als Halbzeuge für die Herstellung von transdermalen therapeutischen Systemen in einem nachfolgenden Produktionsprozess dienen. Die Vorrichtung weist eine (in der Figur nicht dargestellte) Aufnahme für eine Trägerfolienrolle 1' auf, von der die Trägerfolie 1 über eine Beschichtungseinrichtung 7 geführt wird, die eine oder mehrere Bahnen eines typischerweise in einem Lösemittel gelösten, wirkstoffhaltigen Matrixmaterials 2 auf eine der beiden Seiten der Trägerfolie 1 aufträgt. Die Randbereiche der Trägerfolie 1 werden durch den Auftrag nicht beschichtet. Die wie beschrieben beschichtete Trägerfolie durchläuft anschließend eine Heizeinrichtung 8, in der das Lösemittel praktisch vollständig, d.h. bis auf physiologisch unbedenkliche eventuelle Restmengen, aus der Beschichtung ausdampft. In der nachfolgenden Beschreibung wird der Begriff "Matrixmaterial" sowohl für ein in einem Lösemittel gelöstes, als auch für ein von Lösemitteln befreites Matrixmaterial verwendet.

Nach Durchlaufen der Trocknungseinrichtung wird auf die Beschichtung 2 eine Abdeckfolie 3 aufkaschiert. Die Abdeckfolie 3 wird von einer in der Vorrichtung 10 angeordneten Abdeckfolienrolle 3' abgerollt und über eine Kaschiereinrichtung 6 auf die Beschichtungsseite der beschichteten Trägerfolie aufgebracht. Die Breite der Abdeckfolie entspricht bei bevorzugten Ausführungsformen der der Trägerfolie, wobei beide Folien in diesem Fall zweckmäßig deckungsgleich übereinander angeordnet werden, so dass die von der Folienverbindungseinrichtung 9 bewirkte Verbindung der Trägerfolie mit der Abdeckfolie sich beidseits des Matrixmaterials bis zu den Rändern des Laminats 5 erstrecken und die Breite der Matrixbeschichtung somit maximal gewählt werden kann. Eine entsprechende Ausführung ist jedoch nicht zwingend notwendig. Ausführungsformen umfassen daher auch Ausgestaltungen, bei denen Trägerfolie und Abdeckfolie ungleich breit sind und/oder quer zu der durch den in Figur 1 gezeigten Pfeil gekennzeichnete Laufrichtung der Folien versetzt aufeinander angebracht werden. Bei all diesen Ausgestaltungen ist die bahnförmige Matrixmaterialbeschichtung jedoch stets so angeordnet, dass sie beidseits von beiden Folien überlappt wird.

Die Darstellung von Figur 2 zeigt eine schematisierte Draufsicht auf eine zur einbahnigen Beschichtung der Trägerfolie 1 ausgebildete Vorrichtung 10 gemäß Figur 1. Die Darstellung von unterhalb der Trägerfolie 1 gelegener Komponenten dient lediglich der verständlichen Darstellung der Erfindung und soll insbesondere nicht suggerieren, dass die jeweiligen Komponenten zwingend transparent oder durchsichtig sind. Wie Figur 2 zu entnehmen ist, trägt die Beschichtungseinrichtung 7 auf die von der Trägerfolienrolle 1' abgehende Trägerfolie 1 bahnförmig ein Matrixmaterial 2 auf. Die Matrixbahn 2 weist eine geringere Breite als die Trägerfolie 1 auf und ist so auf dieser angeordnet, dass die Trägerfolie 1 zu beiden Seiten über die Ränder der Matrixbahn 2 hinausreicht. Nach dem Durchlaufen der Trocknungseinrichtung 8 und dem Aufkaschieren der Abdeckfolie 3 mithilfe der Kaschiereinrichtung 6 werden die Randbereiche beider Folien 1 und 3 im außerhalb der Matrixbahn 2 gelegenen Bereich miteinander verbunden. Die Verbindung kann stoffschlüssig oder formschlüssig erfolgen. Sie kann bezogen auf den jeweiligen Laminatrand zusammenhängend ausgebildet oder aus einer Vielzahl inselförmiger Verbindungsstellen aufgebaut sein. Auch Kombinationen hiervon sind möglich, beispielsweise eine Verbindung in Form einer sich in Laufrichtung des Laminats erstreckenden durchgehenden Linie mit neben der Linie angeordneten inselförmigen Verbindungsstellen. Allen Ausführungsformen der in Figur 2 schraffiert gekennzeichneten Folienverbindungen 4 ist jedoch gemeinsam, dass das getrocknete Matrixmaterial auch dann nicht durch die Verbindung hindurchtreten kann, wenn es kaltfließende Eigenschaften aufweist. Bei Verwendung von ausschließlich inselförmigen Verbindungsstellen sind die Abstände zwischen diesen Stellen so zu wählen, dass ein Hindurchtreten des Matrixmaterials zwischen den Verbindungsstellen ausgeschlossen ist. Größe und Form der zwischen den Verbindungsstellen gelegenen Bereiche sind somit unter Berücksichtigung der Benetzbarkeit der Folien mit dem Matrixmaterial und dessen Viskosität zu wählen.

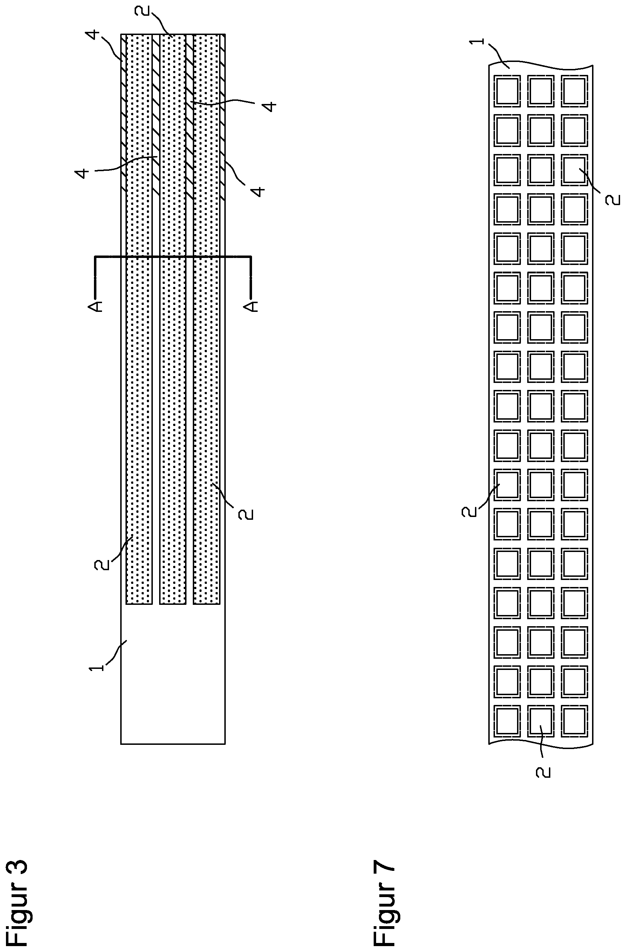

In Figur 3 ist eine schematische Darstellung einer mehrbahnigen Beschichtung der Trägerfolie 1 durch eine wie zuvor beschriebene Vorrichtung in einer Draufsicht gezeigt. Anders als bei der in Figur 2 veranschaulichten Ausführungsform trägt die Beschichtungseinrichtung 7 das Matrixmaterial 2 bei dieser Ausführungsform in mehreren parallel nebeneinander und zueinander beabstandet angeordneten Bahnen 2 auf.

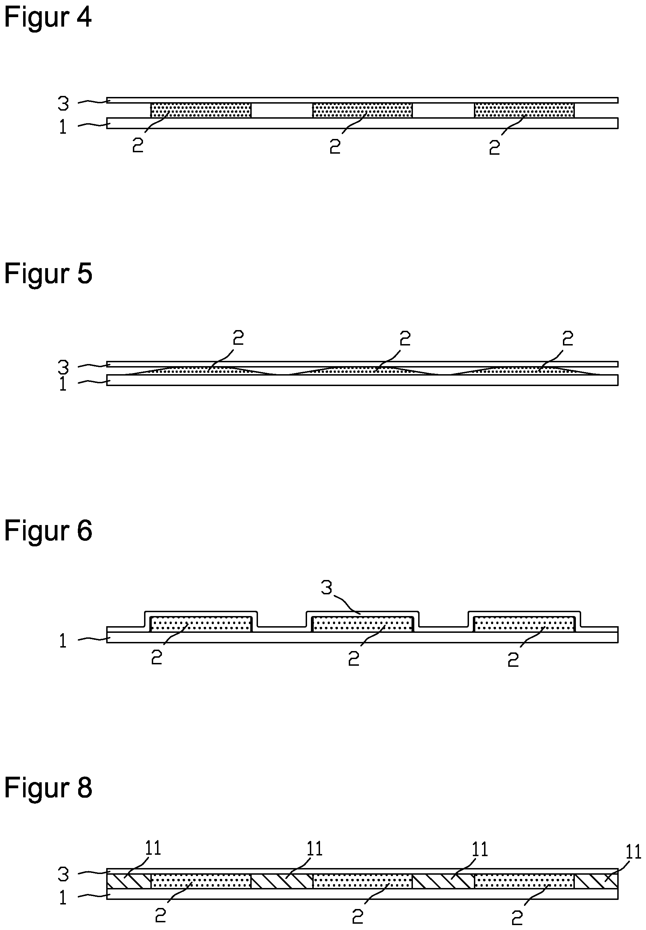

Eine schematisierte Darstellung eines Querschnitts durch das Laminat 5 vor dem Verbinden von Trägerfolie 1 und Abdeckfolie 3 ist in Figur 4 gezeigt. Die Größenverhältnisse der einzelnen Komponenten sind in dieser wie auch in den anderen Figuren nicht maßstabsgetreu, sondern im Hinblick auf eine übersichtliche Darstellung davon abweichend gewählt. Figur 4 veranschaulicht Form und Lage der einzelnen, zwischen Trägerfolie 1 und Abdeckfolie 3 eingebetteten Matrixbahnen 2 unmittelbar nach dem Auftragen. Die Kanten der einzelnen Matrixbahnen weisen die mit dem Herstellungsprozess erzielbare maximale Steigung auf. Bis auf einen vernachlässigbaren Kantenbereich weisen die Matrixbahnen eine gleichmäßige Höhe auf, d. h. sie sind gleichmäßig dick.

Figur 5 veranschaulicht die sich im Laufe der Zeit durch kalten Fluss einstellende Formänderung der Matrixbahnen 2: das Matrixmaterial verfließt, wodurch einerseits der Abstand zwischen den Rändern einander benachbarter Matrixbahnen zunehmend kleiner wird und eventuell sogar verschwindet, andererseits die Dicke der Matrixbahnen, zunächst an den Rändern und schließlich insgesamt, abnimmt.

Die Verbreiterung der Matrixbahnen kann dazu führen, dass bei einem späteren Ausstanzen von Matrixauflagen aus den einzelnen Bahnen nur mehr ein Teil des ursprünglichen Matrixmaterials erfasst wird. Erstreckt sich der Stanzbereich über die ursprüngliche Matrixbahnbreite hinaus, so weist die auf diese Weise erhaltene Matrixauflage eine größere Auflagefläche als vorgesehen auf, mit der Folge, dass sich die vorgesehene Abgabemenge des Wirkstoffes ändert. Ebenso weicht die Wirkstoffabgabe eines unter Verwendung einer solchen "zerflossenen" Matrixbahn erstellten transdermalen therapeutischen Systems von der gewünschten Wirkstoffabgabe ab und stellt somit die therapeutische Eignung eines solchen TDS in Frage, da die durchschnittliche Dicke einer Matrixbahn mit deren Verbreiterung abnimmt.

Um dem abzuhelfen, kann die Trägerfolie mit Vertiefungen ausgebildet sein, in denen das Matrixmaterial aufgenommen wird, und deren Ränder eine Barriere gegen ein Ver- bzw. Zerfließen des Matrixmaterials über die Vertiefungen hinaus bilden. Eine Trägerfolie mit Vertiefungen kann z.B. durch Tiefziehen oder Prägen einer glatten Folie hergestellt werden, deren effektive Dicke sich dadurch jedoch erhöht. Ferner muss die Beschichtungseinrichtung 7 zu den Vertiefungen justiert werden, wobei die Abmaße der Vertiefungen nicht wesentlich größer als die des Beschichtungsauftrags gewählt sein sollten, wenn die Flächenkonzentration des Matrixmaterials zeitlich konstant bleiben soll.

Vorzugsweise werden daher Trägerfolie 1 und Abdeckfolie 3 in den Bereichen außerhalb der Matrixbahnen formschlüssig miteinander verbunden. Dies ist in der schematischen Querschnittsdarstellung von Figur 6 illustriert, wobei darauf hinzuweisen ist, dass die Höhen bzw. Dicken der einzelnen Komponenten, d. h. von Trägerfolie 1, Abdeckfolie 3 und Matrixmaterial 2, stark überhöht dargestellt sind. In der Praxis weisen die für Trägerfolien und Abdeckfolien üblicherweise verwendeten Folien Stärken von 2 bis etwa 200 µm, in einigen Fällen auch bis 500 µm auf. Die lateralen Abmessungen einer Matrixbeschichtung liegen üblicherweise im Zentimeterbereich, während deren Dicke in der Regel nur eine hundert Mikrometer beträgt. Somit kann der Verbindungsbereich zwischen den Folien nahezu direkt an die Ränder einer Matrixbeschichtung anschließend ausgebildet werden.

Bisher wurde die Herstellung von Laminaten beschrieben, bei denen die Matrixbeschichtung in einer oder mehreren Bahnen auf eine Trägerfolie aufgebracht und mit einer Abdeckfolie bedeckt wird. Zur Herstellung von transdermalen therapeutischen Systemen aus solchen Laminaten werden diese bei Verfahren so gestanzt, dass auf der nicht mitgestanzten Trägerfolie oder Abdeckfolie vereinzelte Matrixkerne ausgebildet werden. Der die vereinzelten Matrixkerne umgebende Teil der durchstanzten Lagen des Laminats, das sogenannte Stanzgitter, wird schließlich von der nicht gestanzten Folie abgezogen. Die auf den einzelnen Matrixinseln der Matrixkerne verbliebenen Folienbedeckungen werden bei einigen Verfahren entfernt, beispielsweise mithilfe eines Saughebers. Anschließend werden die Matrixinseln mit einer Backingfolie kaschiert und die transdermalen therapeutischen Systeme durch Stanzen vereinzelt. Vor dem Vereinzeln der einzelnen TDS kann die Backingfolie noch in eine von der Randgeometrie der transdermalen therapeutischen System abweichende Gestalt gestanzt werden.

Die im Schichtverbund eines TDS der Backingfolie gegenüberliegende Folie dient als Schutzfolie, die vor einer Applikation des Systems abgezogen wird. Zur Erleichterung des Abziehens weist die Schutzfolie an der an die Matrix grenzenden Seite vorteilhaft eine Siliconbeschichtung, Polyethylenbeschichtung, Fluorsiliconbeschichtung oder Polytetrafluorethylenbeschichtung auf. Bei anderen Verfahren werden die Matrixkerne von der sie tragenden Folie entfernt und auf eine abziehbare Schutzfolie überführt.

Bei einigen Ausführungsformen wird vor dem Stanzen und vor dem Abziehen des Stanzgitters eine der beiden Folien abgezogen. Um das Abziehen von einer der beiden Folien bzw. des zuvor erwähnten Stanzgitters zu ermöglichen, ist die Festigkeit der Verbindung von erster und zweiter Folie bei Ausführungsformen geringer als die Materialfestigkeit von sowohl der Träger-, als auch der Abdeckfolie. Vorzugsweise ist die Festigkeit der Verbindung der beiden Folien so gewählt, dass die zum Trennen der Verbindung aufzuwendenden Kräfte zu keiner merklichen elastischen oder gar plastischen Verformung von einer der beiden Folien führen.

Bei alternativen Verfahren werden die Folienverbindungen vor einem Trennen der Folien bzw. Abnehmen eines Stanzgitters z. B. durch Schneiden entfernt. In diesem Fall kann eine Folienverbindung mit höherer Festigkeit verwendet werden.

Bei einem anderen Verfahren zur Herstellung von Laminaten werden auf die Trägerfolie keine Matrixbahnen, sondern einzelne Matrixinseln aufgebracht. Als Trägerfolie kann beispielsweise eine Backingfolie mit einer aus dem Bereich von 2 bis 15 µm ausgewählten Dicke verwendet werden, die später die Rückschicht der transdermalen therapeutischen Systeme bildet. Bevorzugt basiert die Backingfolie auf einem Polymer ausgewählt aus der Gruppe bestehend aus Polyolefinen, Olefin-Copolymerisaten, Polyestern, Co-Polyestern, Polyamiden, Co-Polyamiden, Polyurethanen, und dergleichen mehr. Als Beispiele für geeignete Materialien können Polyester und hiervon insbesondere Polyethylenterephthalate als auch Polycarbonate genannt werden, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Copolymerisate wie beispielsweise Acrylnitril-Butadien-Styrol-Terpolymere oder Ethylen-Vinylacetat-Copolymerisate. Auf die Trägerfolie wird zunächst eine (in den Figuren nicht dargestellte) Schablonenfolie aufgebracht, die an den Stellen Öffnungen aufweist, an denen später die Matrixinseln auf der Trägerfolie 1 angeordnet sein sollen. Anschließend wird das wirkstoffhaltige Matrixmaterial 2 gleichmäßig auf die Schablonenseite des Verbunds aus Trägerfolie 1 und Schablonenfolie aufgebracht, so dass die Trägerfolie durch die Öffnungen in der Schablonenfolie hindurch selektiv mit dem Matrixmaterial beschichtet wird. Die Beschichtung kann wie auch bei den zuvor beschriebenen Verfahren in bekannter Weise, z. B. unter Verwendung eines Schlitz- oder Messergießers, erfolgen. Nach dem anschließenden Abziehen der Schablonenfolie verbleiben an den Stellen der Trägerfolie Inseln aus Matrixmaterial 2, an denen sich zuvor die Öffnungen der Schablonenfolie befanden. Eine entsprechende Anordnung von Matrixinseln 2 auf einem Trägerfolienabschnitt 1 ist in der schematischen Draufsicht von Figur 7 dargestellt. In der Darstellung sind nur einige der Matrixinseln mit dem Bezugszeichen 2 versehen.

Im Unterschied zu einem bahnförmigen Auftrag des Matrixmaterials kann das Matrixmaterial bei inselförmigem Auftrag in alle Flächenrichtungen und damit schneller auf größere Fläche zerfließen. Um dem zu begegnen, wird auch bei dieser Ausführungsform die mit Matrixmaterial beschichtete Seite der Trägerfolie mit einer Abdeckfolie kaschiert und beide Folien werden anschließend im Bereich zwischen den Matrixinseln stoffschlüssig oder formschlüssig miteinander verbunden, wobei beide Folien nahe den Matrixrändern vorzugsweise flächig aneinander anschließen. Die Verbindungsstellen können die Matrixinseln bei Ausführungsformen linienförmig umgeben. Ein Beispiel für solche linienförmigen Verbindungsstellen ist in Figur 7 mithilfe der gestrichelten Linien veranschaulicht. Wird als Abdeckfolie eine abziehbare Schutzfolie verwendet, dann können aus dem Laminat transdermale therapeutische Applikationssystem durch Stanzen der Backingfolie, Abziehen des Backingfolienstanzgitters von der Schutzfolie und Vereinzeln der transdermalen therapeutischen Systeme durch Stanzen oder Schneiden der Schutzfolie erstellt werden. Im Hinblick auf ein Ermöglichen von langen Lagerzeiten für die transdermalen Systeme, erfolgt das Stanzen der Backingfolie vorzugsweise so im Verhältnis zu den die Matrixränder umgebenden Verbindungsstellen, dass die Backingfolie außerhalb der Matrix mit der Schutzfolie so verbunden bleibt, dass die Matrix nicht zerfließen kann.

Bei sehr dicken Matrices oder steifen Folien können, wie in Figur 8 veranschaulicht ist, in die Räume zwischen den Matrixbahnen oder Matrixinseln Zwischenfolienstreifen 11 oder Zwischenfoliengitter 11 eingelegt werden, deren Dicke vorzugsweise der Dicke der Matrices entspricht. Die Verbindung von Träger- und Abdeckfolie erfolgt in diesen Fällen vermittelt über die Zwischenfolie(n), d. h., die Trägerfolie wird mit einer der beiden Seiten der Zwischenfolie und die Abdeckfolie mit der anderen der beiden Seiten der Zwischenfolie verbunden. Es sei darauf hingewiesen, dass die Begriffe "Trägerfolie" und "Abdeckfolie", wenn auch mit Bezug auf die Herstellung von Laminaten als Ausgangsbasis für die Herstellung einzelner transdermaler therapeutischer Systeme beschrieben, in dieser Schrift allgemein als ein Matrixmaterial zwischen sich aufnehmende Folien aufzufassen sind, und je nach Anwendungsfall beispielsweise auch stellvertretend für eine Rückschichtfolie bzw. Schutzfolie stehen können.

Die stoffschlüssige Verbindung erfolgt durch Schweißverbindungen, da der Verbindungsbereich geschweißter Folien im Gegensatz zu vielen Klebeverbindungen wirkstoffundurchlässig ist. Zur Herstellung der Schweißverbindungen werden insbesondere Technologien bevorzugt, bei denen die Folien nicht durchgängig erhitzt werden, da die hierbei eingebrachte Wärmemenge zu einer chemischen Veränderung des benachbarten Matrixmaterials und vor allem auch der darin enthaltenen Wirkstoffe führen könnte.

Für das Verbinden von Träger- und Abdeckfolie können bekanntlich Ultraschallschweißverbindungen verwendet werden. Ultraschallschweißverfahren erlauben eine kontinuierliche als auch diskontinuierliche Ausbildung von Schweißverbindungen. Beim Ultraschallschweißen werden mechanische Schwingungen auf die Folien übertragen. Üblich sind Schwingungsfrequenzen aus dem Bereich von etwa 20 bis etwa 35 kHz. Über Molekular- und Grenzflächenreibung wird ein Teil der eingeleiteten mechanischen Schwingungsenergie in Wärme umgesetzt, wodurch das Kunststoffmaterial an den Stellen größter Reibung schließlich zu erweichen beginnt. Da der Dämpfungsfaktor des Kunststoffmaterials mit zunehmender Erweichung ebenfalls zunimmt, konzentriert sich die Materialaufheizung auf die Gebiete mit der größten Reibung, d. h. den sich berührenden Grenzflächen der Folien. Die Konzentration der Ultraschallschwingungen auf die Fügezone der Kunststofffolien kann durch geeignete Gestaltung des Ultraschallkopfes optimiert werden.

Für den Verbindungsvorgang wird nur so viel Wärmeenergie erzeugt, wie zum Plastifizieren der Fügezone erforderlich ist, so dass das wirkstoffhaltige Matrixmaterial 2 durch das Ultraschallschweißen der Folien nicht beeinträchtigt wird. Zur Unterstützung eines geringen Wärmeeintrags weisen die Ultraschallschweißverbindungen quer zu ihrer Längsrichtung vorzugsweise Werte aus dem Bereich von 0,25 bis 3 mm und insbesondere aus dem Bereich von 0,25 bis 1 mm auf. Die Ultraschallschweißverbindungen sind vorzugsweise linienförmig ausgebildet, können bei Ausführungsformen jedoch auch von einer Anordnung inselförmiger Verbindungsstellen gebildet sein. In diesem Fall sind die Verbindungsstellen so nahe zueinander benachbart, dass in den nicht verschweißten Zonen zwischen ihnen kein Matrixmaterial oder ein aus der Matrix austretendes Material hindurchtreten kann. Der Vorteil von inselförmigen Verbindungsstellen wie beispielsweise punktförmigen Verbindungsstellen besteht in der zur insgesamt erzeugten Verbindungsfläche geringeren Schweißverbindungsfläche, die eine Anpassung der zum Trennen der Verbindung erforderlichen Trennkraft an den jeweiligen Anwendungsfall ermöglicht. Um dennoch ein Austreten von sehr dünnflüssigen Matrixkomponenten zu verhindern, kann die Schweißverbindung zur Matrixkante hin eine sehr dünne, als durchgehende Linie ausgebildete Schweißnaht aufweisen, die bei Bedarf leicht aufzutrennen ist. Da diese linienförmige Schweißnaht lediglich als Stopper für dünnflüssige Matrixmaterialien oder -komponenten dient, kann sie auch mit Breiten von unter 0,25 mm ausgeführt sein.

Bei der, falls erforderlich experimentell einzustellenden, Festigkeit der Schweißverbindung ist zu beachten, dass der zu deren Trennung erforderliche Zug, also die Trennkraft pro Flächeneinheit, nicht geringer ist als der durch das Eigengewicht der Komponenten des Verbundes auf die formschlüssige Verbindung ausgeübte Zug, damit die Schweißverbindung nicht durch das Eigengewicht der Laminatkomponenten aufgetrennt werden kann.

Der zum Schweißen verwendete Energieeintrag kann so gewählt werden, dass innerhalb der Schweißnaht eine Bruchstelle entsteht, die sich entlang deren Längsausdehnung erstreckt. Handelt es sich bei der Schweißnaht um eine geschlossene Linie, kann der von der Schweißnaht umschlossene Bereich aus der Laminat-Bahn "herausgedrückt" werden. Ein gebrauchsfertiges TDS, bei dem die Ränder der Trägerfolie mit den Rändern der Abdeckfolie verschweißt sind, kann somit ohne Stanzen durch gezieltes Schweißen eines Laminats hergestellt werden. Wegen des höheren Energieeintrags ist dieses Verfahren nur bei hitzeresistenten Wirkstoffen anwendbar.

Eine ebenfalls bekannte Alternative zur Ultraschallverschweißung stellen Transmissions-Laserschweißverfahren dar, bei denen mithilfe eines NIR-Lasers (Laser mit einer Emissionswellenlänge im nahen Infrarotbereich) Material an den Kontaktflächen von Trägerfolie und Abdeckfolie erweicht wird. Die Verschweißung der Folien an den Kontaktflächen kann durch Druckeinwirkung unterstützt werden, der über ein für das NIR-Laserlicht durchsichtiges und von diesem durchstrahltes Werkstück auf die Schweißbereiche ausgeübt wird.

Um nur den Bereich an den sich berührenden Oberflächenbereichen der Folien zu plastifizieren, muss der Energieeintrag in die vom Laser durchstrahlte Folie minimal sein. Daher ist wenigstens die dem einstrahlenden Laser zugewandte Folie aus einem das Laserlicht praktisch nicht absorbierenden Material gefertigt. Besteht die andere Folie aus einem das Laserlicht absorbierenden Material, dann plastifiziert das Laserlicht dessen an der Kontaktfläche bestrahlte Oberfläche und hierüber die Kontaktfläche der das Laserlicht nicht absorbierenden Folie, wodurch die beiden Folien in den plastifizierten Bereichen stoffschlüssig miteinander verbunden werden können.

Sind beide Folien 1 und 3 aus einem das Laserlicht nicht absorbierenden Material gefertigt, so wird die Kontaktfläche von wenigstens einer der beiden Folien an der Kontaktseite mit einer dünnen, das Laserlicht absorbierenden Beschichtung versehen. Statt nur einer Folie können auch die Kontaktbereiche beider Folien entsprechend beschichtet werden. Die Absorberschicht absorbiert das Licht des zum Schweißen verwendeten Lasers und plastifiziert hierdurch die aneinander angrenzenden Oberflächenbereiche der beiden sich an den Kontaktflächen berührenden Folien, wodurch eine stoffschlüssige Verbindung hergestellt werden kann.

Als Laser eignen sich beispielsweise Diodenlaser mit Emissionswellenlängen im Bereich von 808 bis 980 nm und Nd:YAG-Laser mit einer Emissionswellenlänge von 1064 nm. Vorzugsweise werden Laser mit Emissionen im Bereich von 940 bis 1064 nm verwendet. Als Absorbermaterialien werden bekannte Stoffe verwendet, wie sie z.B. von Crysta-Lyn Chemical Corporation unter der Markenbezeichnung Clearweld® vertrieben werden.

Bei dem beschriebenen Transmissionsschweißverfahren wird nur ein dünner Oberflächenbereich an den einander berührenden Folienoberflächen plastifiziert. Die Temperaturen unterhalb bzw. oberhalb der plastifizierten Zone sind stets niedriger als die Glasübergangstemperatur der Folienpolymere, so dass die strukturelle Integrität der Folien durch den Schweißvorgang nicht beeinträchtigt wird. Aufgrund des geringen Energieeintrags und der lokal begrenzten Schweißzone, ist die Beeinträchtigung der Folien durch den Schweißvorgang wie beim Ultraschallschweißen vernachlässigbar.

Bei Verwendung einer Zwischenfolie zum Verschweißen von Trägerfolie und Abdeckfolie werden vorzugsweise beide Kontaktflächen, d. h. die Kontaktfläche der Zwischenfolie mit der Trägerfolie und die lateral an der derselben Stelle gelegene Kontaktfläche der Zwischenfolie mit der Abdeckfolie gleichzeitig verschweißt. Um dies zu erreichen, ist die Auftragsdicke der Absorberschicht vorzugsweise so an deren Absorptionsvermögen für das NIR-Laserlicht abgestimmt, dass das Laserlicht die ihm zugewandte erste Absorberschicht teilweise durchdringt und der durch die erste Absorberschicht hindurchgetretene Teil des Laserlichts in der zweiten Absorberschicht zumindest teilweise absorbiert wird. Die an den beiden Kontaktflächen verwendeten Absorbermaterialien können identisch, aber auch verschieden sein. Vorzugsweise sind sie so gewählt, dass in jeder der beiden Kontakt- bzw. Schweißbereiche in etwa dieselbe Lichtleistung absorbiert wird.

Als formschlüssige Verbindungstechniken werden Prägetechniken bevorzugt, die die Folien im Verbindungsbereich so verformen, dass die in die eine der beiden Folien eingeprägten Musterelemente zur Aufnahme in den Musterelementen ausgebildet sind, die in die andere der beiden Folien eingeprägt werden. Durch den Formschluss der Musterelemente wird ein Verschieben der beiden Folien lateral zueinander unterbunden. Um ein Trennen der Folien durch einen senkrecht zu deren lateraler Ausdehnung wirkenden Zug zu verhindern, können die Musterelemente auch zu einem Formschluss senkrecht zu dieser Richtung ausgebildet sein. Da die Trennkraft ausreichend gering sein soll, damit die Folien beim Auftrennen der Verbindung nicht einreißen, ist ein entsprechender Formschluss der Prägemusterelemente auch senkrecht zur lateralen Ausdehnung nicht zwingend erforderlich. In der Regel ist die Trennkraft ausreichend hoch, um ein unbeabsichtigtes Auftrennen der Formschlussverbindung zu verhindern, wenn die ineinander gefügten Prägemusterelemente kraftschlüssig miteinander verbunden sind. Ein entsprechender Kraftschluss wird bei vorteilhaften Ausführungsformen mit einem Rändelverfahren realisiert, bei dem die Folien bezüglich ihrer lateralen Richtungen formschlüssig und senkrecht hierzu kraftschlüssig verbunden sind. Die Stärke des Kraftschlusses wird über die Druckkraft beim Rändeldrücken bestimmt und wird wenigstens so stark gewählt, dass der zum Trennen der Folien senkrecht zu deren lateraler Ausdehnung erforderliche Zug größer ist als der durch das Eigengewicht der Komponenten des Verbundes auf die formschlüssige Verbindung ausgeübte Zug.

Statt Trägerfolie und Abdeckfolie direkt formschlüssig miteinander zu verbinden, kann die formschlüssige Verbindung auch mithilfe einer zwischen den beiden Folien angeordneten Zwischenfolie realisiert werden. Abhängig davon, ob die wirkstoffhaltige Matrix bahn- oder inselförmig ausgebildet ist, wird vorzugsweise eine streifen oder gitterförmige Zwischenfolie verwendet, in der Prägemuster ausgebildet sind, die entweder in entsprechend an den Folien ausgebildete Prägemuster eingreifen oder in die entsprechend an den Folien ausgebildete Prägemuster eingreifen. Die Verwendung von Zwischenfolien ist insbesondere in Verbindung mit dicken Matrices aber auch bei steifen Folien von Vorteil, da sie einem Zusammendrücken der Matrix und einer damit verbundenen Kissenbildung, bei der sich das Flächengewicht der Matrix mit dem Abstand zu deren Randzone verändern würde, wirksam vorbeugt.

Sowohl bei einer direkten als auch bei einer über eine Zwischenfolie vermittelten formschlüssigen Verbindung, liegen die Folien im oder benachbart zum Verbindungsbereich so flächig aufeinander, dass kein Matrixmaterial bzw. Komponenten hiervon durch den Verbindungsbereich fließen können.

Die formschlüssige Verbindung von Trägerfolie und Abdeckfolie ist insbesondere bei sehr temperaturempfindlichen Matrixmaterialien von Vorteil, da sie mit keinem kritischen Wärmeeintrag verbunden ist.

Die Erfindung ermöglicht die Herstellung von langzeitlagerfähigen transdermalen therapeutischen System sowie Halbzeugen zu deren Herstellung, die kalt fließende Matrixmaterialien enthalten.

Die vorliegende Erfindung ermöglicht insbesondere ein gebrauchsfertiges TDS, bei dem die Rückschichtfolie und die Schutzfolie mindestens teilweise über die Ränder der Matrix hinausreichen und in den Bereichen außerhalb der Matrix mindestens teilweise stoffschlüssig oder formschlüssig miteinander verbunden sind. Hierbei erfolgt die stoff- oder formschlüssige Verbindung so, dass die Entfernung der Schutzfolie vor Gebrauch des TDS nicht oder nur unwesentlich behindert wird. Bei einer bevorzugten Ausführungsform ist deshalb die Festigkeit der stoffschlüssigen oder formschlüssigen Verbindung geringer als die Festigkeit der Schutzfolie und die Festigkeit der Rückschichtfolie.

## Patentansprüche

1. Transdermales therapeutisches System mit einer ersten wirkstoffundurchlässigen Folie (1), einer zweiten wirkstoffundurchlässigen Folie (3) und einer zwischen der ersten und der zweiten Folie angeordneten wirkstoffhaltigen Matrix (2), wobei erste und zweite Folie über die Ränder der Matrix hinausreichen und in Bereichen außerhalb der Matrix so formschlüssig miteinander verbunden sind, dass die Matrix die Verbindung (4) von erster und zweiter Folie nicht durchdringen kann, wobei die Festigkeit der Verbindung (4) von erster (1) und zweiter Folie (3) geringer ist als die Materialfestigkeit von sowohl der ersten als auch der zweiten Folie.

2. Laminat zur Herstellung transdermaler therapeutischer Systeme, wobei das Laminat (5) eine erste wirkstoffundurchlässige Folie (1), eine zweite wirkstoffundurchlässige Folie (3) und eine zwischen der ersten und der zweiten Folie angeordnete wirkstoffhaltige Matrix (2) aufweist, wobei sowohl die erste als auch die zweite Folie über die Ränder der Matrix hinausreicht und erste und zweite Folie in Bereichen außerhalb der Matrix so formschlüssig miteinander verbunden sind, dass die Matrix die Verbindung (4) von erster und zweiter Folie nicht durchdringen kann, wobei die Festigkeit der Verbindung (4) von erster (1) und zweiter Folie (3) geringer ist als die Materialfestigkeit von sowohl der ersten als auch der zweiten Folie.

3. Transdermales therapeutisches System oder Laminat nach einem der Ansprüche 1 oder 2, worin die formschlüssige Verbindung (4) von erster (1) und zweiter (3) Folie durch Prägemuster gebildet ist, bei denen die Musterelemente des Prägemusters in einer der beiden Folien (1, 3) so ausgebildet sind, dass sie in entsprechend ausgebildete Prägemusterelemente der anderen der beiden Folien (3, 1) so formschlüssig eingreifen, dass eine Bewegung der Folien lateral zueinander unterbunden ist und der zum Trennen der Folien senkrecht zu deren lateraler Ausdehnung erforderliche Zug größer als der durch das Eigengewicht der Komponenten des Verbundes auf die formschlüssige Verbindung ausgeübte Zug ist.

4. Transdermales therapeutisches System oder Laminat nach Anspruch 3, worin das Prägemuster der ersten Folie und das Prägemuster der zweiten Folie Ergebnis eines Rändelverfahrens sind.

5. Transdermales therapeutisches System oder Laminat nach Anspruch 4, worin die formschlüssige Verbindung (4) entweder als direkter Formschluss von erster und zweiter Folie oder als Formschluss einer zwischen erster und zweiter Folie angeordneten Zwischenfolie (11) mit der ersten (1) und mit der zweiten (3) Folie ausgebildet ist.

6. Verfahren zur Herstellung von Laminaten (5) und/ oder transdermalen therapeutischen Systemen, das folgende Schritte aufweist:
Herstellen eines Verbunds aus einer ersten wirkstoffundurchlässigen Folie (1), einer zweiten wirkstoffundurchlässigen Folie (3) und einer zwischen erster und zweiter Folie angeordneten Schicht aus einem Matrixmaterial (2), wobei die lateralen Abmessungen der Matrixschicht (2) zumindest in einer Richtung kleiner als die von erster (1) und zweiter (3) Folie sind und erste und zweite Folie sich in dieser Richtung über beide Ränder der Matrixschicht hinaus erstrecken,
Verbinden von erster Folie (1) und zweiter Folie (3) in Bereichen außerhalb der Matrix (2) formschlüssig so, dass die Matrix (2) die Verbindung (4) von erster und zweiter Folie nicht durchdringen kann, wobei die Verbindung mit einer Festigkeit ausgeführt wird, die geringer ist als die Materialfestigkeit von sowohl der ersten (1) als auch der zweiten (3) Folie, und die zumindest so groß ist, dass der zum Trennen der Folien senkrecht zu deren lateraler Ausdehnung erforderliche Zug größer als das Flächengewicht des Verbunds aus erster Folie (1), zweiter Folie (3) und Matrix (2) ist.

7. Verfahren nach Anspruch 6, worin das formschlüssige Verbinden von erster Folie (1) und zweiter Folie (3) in Bereichen außerhalb der Matrix (2) mittels Prägedrücken erfolgt.

## Claims

1. A transdermal therapeutic system comprising a first film (1) impervious to active substances, a second film (3) impervious to active substances, and an active substance-containing matrix (2) arranged between the first and second films, wherein the first and second films extend beyond the edges of the matrix and are joined together in areas outside the matrix in an interlocking manner such that the matrix cannot penetrate the joint (4) of the first and second films, the strength of the joint (4) between the first (1) and second (3) films being less than the material strength of both the first and second films.

2. A laminate for the preparation of transdermal therapeutic systems, the laminate (5) comprising a first film (1) impervious to active substances, a second film (3) impervious to active substances, and an active substance containing matrix (2) arranged between the first and second films, wherein the first and second films extend beyond the edges of the matrix and are joined together in an interlocking manner in areas outside the matrix such that the matrix cannot penetrate the joint (4) between the first and second films, the strength of the joint (4) between the first (1) and second (3) films being less than the material strength of both the first and second film.

3. The transdermal therapeutic system or laminate of any of claims 1 or 2, wherein the interlocking joint (4) between the first (1) and second (3) films is formed by embossing patterns with the pattern elements of the embossing pattern in one of the two films (1, 3) designed to engage in correspondingly designed embossing pattern elements of the other of the two films (3, 1) in an interlocking manner for preventing a lateral movement of the films relative to each other with the tensile force required for separating the films in a direction perpendicular to the lateral extension of the films being greater than the tensile force exerted on the interlocking joint by the self-weight of the components of the composite.

4. The transdermal therapeutic system or laminate of claim 3, wherein the embossing pattern of the first film and the embossing pattern of the second film are the result of a knurling process.

5. The transdermal therapeutic system or laminate of claim 4, wherein the interlocking joint (4) is formed either as a direct positive locking of the first and second films or as a positive locking formed by an intermediate film (11) arranged between the first and second films with the first (1) and the second (3) films.

6. A method for manufacturing laminates (5) and/or transdermal therapeutic systems, comprising the following steps:
preparing a composite of a first film (1) impervious to active substances, a second film (3) impervious to active substances, and a layer of a matrix material (2) arranged between the first and second films, wherein the lateral dimensions of the matrix layer (2) are in at least one direction smaller than those of the first (1) and second (3) films, with the first and second films extending in said direction beyond both edges of the matrix layer,
joining the first film (1) to the second film (3) in areas outside the matrix (2) in an interlocking manner such that the matrix (2) cannot penetrate the joint (4) between the first and second films, wherein the joint is provided with a strength smaller than the material strength of both, the first (1) and second (3) films, and at least sufficiently high such that the tensile force required for separating the films in a direction perpendicular to the lateral expansion of the films is greater than the surface weight of the compound formed by the first film (1), the second film (3), and the matrix (2).

7. The method of claim 6, wherein the positive locking of the first (1) and second (3) films in areas outside the matrix (2) is effected by means of embossing.

## Revendications

1. Système thérapeutique transdermique avec une première feuille (1) imperméable au principe actif, une deuxième feuille (3) imperméable au principe actif et une matrice (2) contenant un principe actif disposée entre la première et la deuxième feuille, dans lequel les première et deuxième feuilles recouvrent les bords de la matrice et sont reliées l'une à l'autre dans des zones extérieures à la matrice par complémentarité de forme, de telle sorte que la matrice ne puisse pas traverser la liaison (4) des première et deuxième feuilles, dans lequel la solidité de la liaison (4) des première (1) et deuxième feuilles (3) est plus faible que la solidité du matériau aussi bien de la première que de la deuxième feuille.

2. Stratifié pour la production de systèmes thérapeutiques transdermiques, dans lequel le stratifié (5) présente une première feuille (1) imperméable au principe actif, une deuxième feuille (3) imperméable au principe actif et une matrice (2) contenant un principe actif disposée entre la première et la deuxième feuille, dans lequel aussi bien la première que la deuxième feuille recouvrent les bords de la matrice et la première et la deuxième feuille sont reliées l'une à l'autre dans des zones extérieures à la matrice par complémentarité de forme, de telle sorte que la matrice ne puisse pas traverser la liaison (4) des première et deuxième feuilles, dans lequel la solidité de la liaison (4) des première (1) et deuxième feuilles (3) est plus faible que la solidité du matériau aussi bien de la première que de la deuxième feuille.

3. Système thérapeutique transdermique ou stratifié selon l'une des revendications 1 ou 2, dans lequel la liaison (4) par complémentarité de forme des première (1) et deuxième feuilles (3) est formée par des motifs de gaufrage, dans lesquels les éléments de motif du motif de gaufrage sont réalisés dans l'une des deux feuilles (1, 3) de telle sorte qu'ils soient en prise par complémentarité de forme dans des éléments de motif de gaufrage réalisés de façon correspondante de l'autre des deux feuilles (3, 1), de telle sorte qu'un mouvement des feuilles latéralement l'une par rapport à l'autre soit empêché et la traction nécessaire pour séparer les feuilles, perpendiculairement à leur extension latérale, est supérieure à la traction exercée par le propre poids des composants du composite sur la liaison par complémentarité de forme.

4. Système thérapeutique transdermique ou stratifié selon la revendication 3, dans lequel le motif de gaufrage de la première feuille et le motif de gaufrage de la deuxième feuille sont le résultat d'un procédé de moletage.

5. Système thérapeutique transdermique ou stratifié selon la revendication 4, dans lequel la liaison (4) par complémentarité de forme est réalisée soit en tant que complémentarité de forme directe des première et deuxième feuilles, soit en tant que complémentarité de forme d'une feuille intermédiaire (11) disposée entre les première et deuxième feuilles avec la première (1) et avec la deuxième (3) feuille.

6. Procédé de production de stratifiés (5) et/ou de systèmes thérapeutiques transdermiques qui présente les étapes suivantes :
production d'un composite à partir d'une première feuille (1) imperméable au principe actif, d'une deuxième feuille (3) imperméable au principe actif et d'une couche disposée entre des première et deuxième feuilles d'un matériau de matrice (2), dans lequel les dimensions latérales de la couche de matrice (2) au moins dans une direction sont plus petites que celles des première (1) et deuxième (3) feuilles et les première et deuxième feuilles s'étendent dans cette direction sur les deux bords de la couche de matrice,
liaison de la première feuille (1) et de la deuxième feuille (3) dans des zones extérieures à la matrice (2) par complémentarité de forme de telle sorte que la matrice (2) ne puisse pas traverser la liaison (4) des première et deuxième feuilles, dans lequel la liaison est exécutée avec une solidité qui est plus faible que la solidité du matériau aussi bien de la première (1) que de la deuxième (3) feuille, et qui a au moins une grandeur telle que la traction nécessaire pour séparer les feuilles, perpendiculairement à leur extension latérale, soit supérieure au grammage du composite de la première feuille (1), de la deuxième feuille (3) et de la matrice (2).

7. Procédé selon la revendication 6, dans lequel la liaison par complémentarité de forme de la première feuille (1) et de la deuxième feuille (3) s'effectue dans des zones extérieures à la matrice (2) au moyen du gaufrage.
